# EUROPEAN PATENT SPECIFICATION

(11) **EP 0 693 295 B1**
(45) Date of publication and mention of the grant of the patent: **25.09.2002**
(21) Application number: 95201873.7
(22) Date of filing: 07.07.1995
(51) Int. Cl.: A61M 1/00

(54) **Suction catheter**
Absaugkatheter
Catheter d'aspiration

(30) Priority: 19.07.1994 NL 9401184
(43) Date of publication of application: 24.01.1996
(73) Proprietor: CORDIS EUROPA N.V., NL-9301 LJ Roden (NL)
(72) Inventor: Bosma, Gjalt, NL-9201 EK Drachten (NL); Boudewijn, Alexander Christiaan, NL-9351 KS Leek (NL)
(74) Representative: 't Jong, Bastiaan Jacob

(56) References cited:
- EP-A- 0 442 579
- WO-A-91/05575
- US-A- 4 637 814
- US-A- 5 078 688
- US-A- 5 300 022

## Description

The invention relates to a suction catheter of the type as described in the preamble of claim 1. A catheter of this kind is known from US-A-5300022.

A drawback of this suction catheter is that it tends to buckle quite easily under certain circumstances, as a result of which it ceases to function.

The object of the invention is to provide a suction catheter of this type which does not suffer this problem.

This object is achieved with the suction catheter as characterised in claim 1.

It has become apparent that the unfavourable buckling behaviour of the known catheter is due to the asymmetrical cross-section of the basic body.

With the catheter according to the invention the buckling behaviour is only defined by the basic body. The separate tube-like body lies more or less free inside the lumen of the basic body and does not influence the buckling behaviour. Consequently the catheter according to the invention has a constant bending stiffness in all directions which is conducive to the prevention of buckling behaviour. By using separate tube-like bodies for both the pressure channel and the guide wire channel, the available cross-section of the lumen of the basic body is used most effectively, as a result of which even with small diameters of the suction catheter, there is enough cross-section left to function as discharge channel. Also the second separate tube-like body lies substantially free inside the lumen of the basic body, so that this does not influence the bending performance of the catheter.

A suitable, easy to handle embodiment of the catheter according to the invention is characterised in claim 2.

Preferably both measures as set out in claim 3 are employed. As a result, no separate provision for haemostasis is required, so that a manageable unit is obtained.

By employing the measure as set out in claim 4, the trifurcation can form the extreme proximal part of the catheter which means the catheter does not need to comprise any further tube sections for the connection of the different channels.

A suitable, easy to handle embodiment is characterised in claim 6.

Preferably the measure as set out in claim 7 is employed. The integrated spray nozzle forms a liquid jet pump, as a result of which the suction action in the distal end of the catheter will be reinforced. Thus, even when the basic body has a very small diameter, sufficient suction can be created without the need for an additional suction pump.

A favourable further development is characterised in claim 8. As the pressure channel and the guide wire channel have been formed in separate tube-like bodies, it is not difficult to assemble the basic body from a relatively stiff part and a relatively pliant part during the manufacturing process. Only the basic body parts need to be connected to each other. The other tube-like members extend continuously. Thus a catheter can be made which is capable of reaching more tortuous blood vessels.

The invention will be explained in greater detail in the following description with reference to the attached drawings.

Fig. 1 shows a partly broken away and cross-sectional view of a catheter according to a first embodiment of the invention.

Fig. 2 shows a trifurcation of a catheter according to the invention in a preferred embodiment.

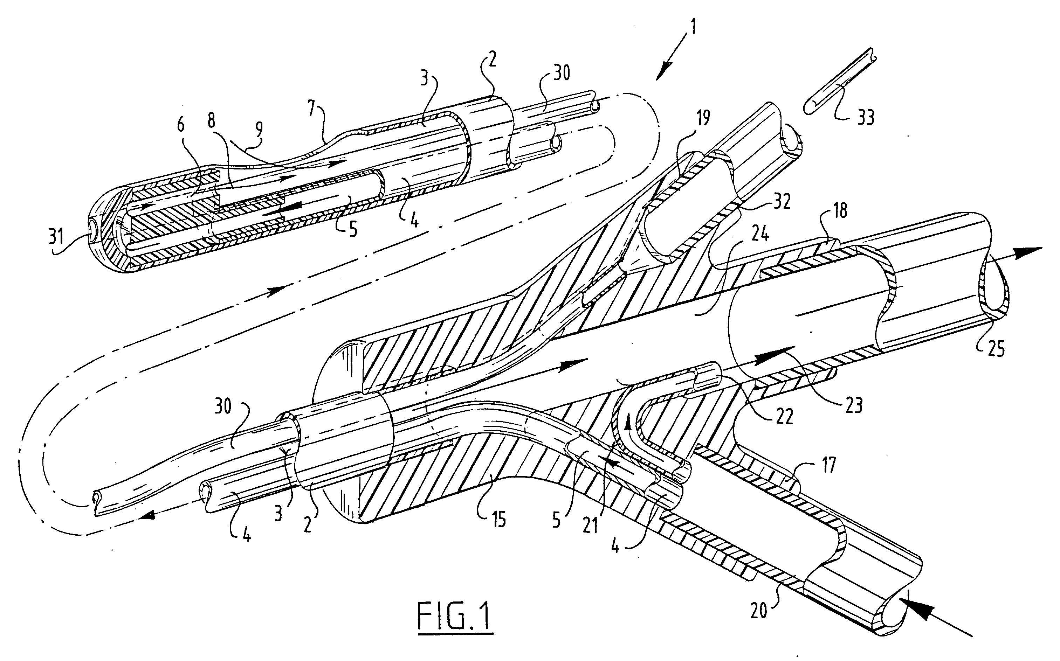

In fig. 1 the distal end and the proximal end of the catheter 1 have been shown. The intermediate section has been indicated with a dashed and dotted line.

The catheter 1 has a tube-like basic body 2 with a substantially circular cross-section. At the proximal end a trifurcation 15 has been formed, in which several channels, to be described later, are brought together. At the distal end an opening 7 has been formed in the basic body 2. This opening 7 forms a suction inlet which is connected with the lumen 3.

In the lumen 3 a first separate tube-like body 4 has been received. It extends from the proximal end to the distal end. In the tube-like body 4 a channel 5 has been defined which comprises a bent back section 6 in the distal end and ends in a jet nozzle. Liquid under pressure, supplied via channel 5 can thus be directed as a liquid jet 8 along the opening 7, as a result of which a suction will be created indicated by arrow 9.

The trifurcation 15 comprises a first branch 17 which forms an inlet for the pressure channel 5. Inside this branch 17 a tube section 20 has been fixed which is connected to a source of liquid under pressure.

A second branch 18 of the trifurcation 15 forms the connection with the lumen 3 and functions as the discharge channel of the catheter. With the branch 18 a tube section 25 is connected which can be connected to a collecting reservoir.

The third branch 19 is also connected with a tube section 32 and forms a connection with a second separate tube-like body 30 inside the lumen 3. This tube-like body 30 forms a channel for a guide wire 33. The tube-like body 30 has been fed through as far as the distal end of the catheter 1 and has been received with its circumference sealed in the rounded front wall of the distal end. Thus the guide wire 33 can extend over the entire length through the catheter and be pushed out of the front of the catheter at the opening 31. The catheter can thus be passed over the guide wire 33.

As can be seen clearly, a branch 21 of the pressure connection 20 has been received in the trifurcation 15. This branch 21 debouches into the discharge channel 24 with a spray nozzle 22 which is directed in the proximal direction. The liquid under pressure supplied via the pressure line 20 flows partly through the branch 21 and forms a liquid jet indicated with arrow 23 which, as seen in fig. 1, is aimed towards the right. Thus a liquid jet pump is formed in the discharge channel 24 which creates additional suction in the channel 24. Even in the case of a small diameter of the basic body 2 and hence a relatively small cross-section of the area of the lumen 3 available for the discharge channel, sufficient suction can be created at the opening 7.

As the tubes 4 and 30 are different members which have been received substantially free in the basic body 2, they influence the bending performance of the basic body at the most to a very limited degree. Because of the symmetrical cross-section of the basic body 2 there is no preferred direction as far as bending is concerned so that the basic body is not sensitive to buckling.

Although not shown in the figure, the catheter according to the invention can in a suitable manner be manufactured in such a way that the basic body 2 will be made up of a relatively bending-stiff proximal section and a relatively pliable distal section. Manufacturing such a catheter is relatively simple as the tube-like bodies 30 and 4 do not need to be interrupted. Only one weld needs to be made between the two tube-like sections making up the basic body 2. A catheter manufactured in this way has consequently a more pliable distal end-section as a result of which more tortuous blood vessels can be reached.

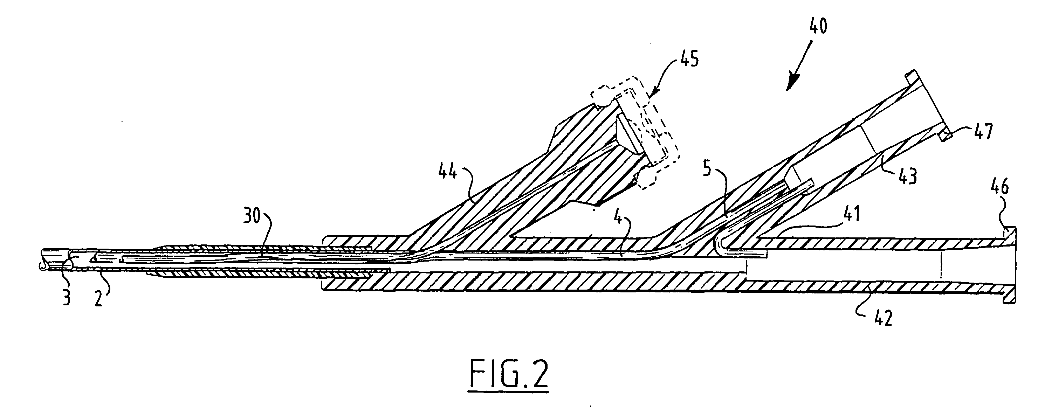

The same reference numbers have been used for corresponding parts in fig. 1 and fig. 2.

Just like trifurcation 15, trifurcation 40 has been attached by means of injection moulding to the basic body 2, in which the tube-like bodies 4 and 30 have already been received.

The basic body 2, made up of the tube-like bodies 4 and 30, is placed in a suitably shaped mould and the protruding tube sections 4 and 30 are placed in the desired position. The required spaces, in particular those for forming the channel sections, are kept open by means of core pins which connect to the tube-like bodies and the basic body. A tube section 41 is also received in the mould and thus embedded in the trifurcation 40. This tube section 41 has the same function as the branch 21 in fig. 1.

As fig. 2 shows, the tree-way furcation 40 has been manufactured in such a way that the discharge channel extends in a straight line to a branch 42. The pressure channel 5 and the guide wire channel in the tube-like body 30, are received in the branches 43 and 44 of the trifurcation 40 respectively, which extend laterally, parallel to each other at an oblique angle away from the discharge channel.

The branch 44 in which the tube-like body 30 has been received, is provided with a, as such known, haemostatic valve 45 at its free end. In this way a guide wire can be introduced and removed directly via the branch 44 of the trifurcation, without a need for any additional haemostatic provisions.

The branch 42 of the discharge channel and the branch 43 of the pressure channel are both provided with male Luer-lock adapters 46 and 47 respectively. Consequently the suction catheter according to the invention provided with the trifurcation 40 has a very compact construction and is easy to handle.

## Claims

1. Suction catheter (1) comprising a tube-like basic body (2) with a distal and a proximal end, with a lumen (3) forming a discharge channel, and a pressure channel (5) formed in a separate tube-like body (4) received inside the lumen (3), wherein at the distal end the pressure channel is connected with a spray nozzle and the discharge channel with a suction inlet and at the proximal end the channels are connected to connecting members, **characterized in that** the basic body (2) in cross-section is substantially symmetrical with respect to a central point thereof over substantially the whole length thereof, wherein inside the lumen (3) of the basic body (2) a second separate tube-like body (30) has been received which forms a channel for a guide wire (33), which second tube-like body (30) at the distal end has been arranged with its circumference sealed in the wall of the basic body (2) and has been provided with an introduction member close to the proximal end.

2. Catheter as claimed in claim 1, comprising a trifurcation (15) at the proximal end with one outlet at the distal side to which the basic body (2) has been connected and three separate outlets at the proximal side for the guide wire channel, the pressure channel (5) and the discharge channel respectively.

3. Catheter as claimed in claim 2, wherein the outlet of the guide wire channel has been provided with a haemostatic valve (45).

4. Catheter as claimed in claim 2 or 3, wherein the outlets of the pressure channel and the discharge channel are provided with Luer-lock adapters (46, 47).

5. Catheter as claimed in claim 3 and 4, wherein the haemostatic valve (45) and the Luer-lock adapters (46, 47) have been integrated in the trifurcation.

6. Catheter as claimed in one of the claims 2 - 5, wherein the trifurcation (40) has been manufactured in such a way that the discharge channel extends in a straight line and the guide wire channel and the pressure channel (5) extend laterally, parallel to each other at an oblique angle away from the discharge channel.

7. Catheter as claimed in one of the claims 2 - 6, wherein a connecting channel (21) between the pressure channel (5) and the discharge channel (3) has been formed in the trifurcation (15) which debouches into the discharge channel (3) by means of a spray nozzle (22) directed in the proximal direction.

8. Catheter as claimed in one of the previous claims, wherein the basic body (2) has been made up of a relatively stiff proximal section and a relatively pliable distal section, and the at least one separate tube-like member consists of one continuous unit.

## Patentansprüche

1. Absaugkatheter (1) mit einem ein distales und ein proximales Ende aufweisenden rohrartigen Grundkörper (2), mit einem einen Abgabekanal ausbildenden Lumen (3) und einem Druckkanal (5), der in einem im Inneren des Lumens (3) aufgenommenen, separaten rohrartigen Körper (4) ausgebildet ist, wobei der Druckkanal an dem distalen Ende mit einer Sprühdüse und der Abgabekanal mit einem Absaugeinlaß verbunden sind und die Kanäle am proximalen Ende mit Verbindungselementen verbunden sind, **dadurch gekennzeichnet, daß** der Grundkörper (2) im Querschnitt im wesentlichen symmetrisch in Bezug auf einen seiner Mittelpunkte über im wesentlichen seine gesamte Länge ist, wobei im Inneren des Lumens (3) des Grundkörpers (2) ein zweiter separater, rohrartiger Körper (30) aufgenommen ist, der einen Kanal für einen Führungsdraht (33) bildet, wobei der zweite rohrartige Körper (30) an dem distalen Ende mit seinem Umfang in der Wand des Grundkörpers (2) abgedichtet, angeordnet ist und mit einem Einführelement, nahe dem proximalen Ende, versehen ist.

2. Katheter nach Anspruch 1, der eine Gabelung (15) in drei Zweige an dem proximalen Ende aufweist, mit einem Auslaß an dem distalen Ende, an dem der Grundkörper (2) angeschlossen ist und drei separaten Auslässen an der proximalen Seite für den Führungsdrahtkanal, den Druckkanal (5) bzw. den Abgabekanal.

3. Katheter nach Anspruch 2, wobei der Auslaß des Führungsdrahtkanals mit einem hämostatischen Ventil (45) versehen ist.

4. Katheter nach Anspruch 2 oder 3, wobei die Auslässe des Druckkanals und des Abgabekanals mit Luer-Lockadaptern (46, 47) versehen sind.

5. Katheter nach Anspruch 3 und 4, wobei das hämostatische Ventil (45) und die Luer-Lockadapter (46, 47) in die Gabelung in drei Zweige integriert sind.

6. Katheter nach einem der Ansprüche 2 bis 5, wobei die Gabelung (40) derart hergestellt ist, daß sich der Abgabekanal in einer geraden Linie erstreckt und der Führungsdrahtkanal und der Druckkanal ((5) sich seitlich, parallel zueinander unter einem geneigten Winkel, weg von dem Abgabekanal, erstrecken.

7. Katheter nach einem der Ansprüche 2 bis 6, wobei ein Verbindungskanal (21) zwischen dem Druckkanal (5) und dem Abgabekanal (3) in der Gabelung (15) in drei Zweige ausgebildet ist, der über eine in die proximale Richtung gerichtete Sprühdüse (22) in den Abgabekanal (3) mündet.

8. Katheter nach einem der vorstehenden Ansprüche, wobei der Grundkörper (2) hergestellt ist aus einem relativ steifen proximalen Abschnitt und einem relativ biegsamen distalen Abschnitt und das wenigstens eine separate, rohrartige Element aus einer durchgehenden Einheit besteht.

## Revendications

1. Cathéter d'aspiration (1) comprenant un corps de base (2) en forme de tube qui présente une extrémité distale et une extrémité proximale, un lumen (3) formant un canal de drainage, et un canal de pression (5) formé dans un corps séparé (4) en forme de tube reçu à l'intérieur du lumen (3), cathéter dans lequel, à l'extrémité distale, le canal de pression est relié à une buse de pulvérisation et le canal de drainage est relié à une entrée d'aspiration, et à l'extrémité proximale, les canaux sont reliés à des organes de liaison, **caractérisé en ce que** le corps de base (2) est, en section transversale, sensiblement symétrique par rapport à un point central dudit corps sur sensiblement toute la longueur du corps de base, et **en ce qu'**à l'intérieur du lumen (3) du corps de base, un deuxième corps séparé (30) en forme de tube a été reçu et forme un canal pour un câble de guidage (33), ledit deuxième corps (30) en forme de tube, au niveau de son extrémité distale, ayant été prévu avec sa circonférence fixée de façon étanche dans la paroi du corps de base (2) et ayant été prévu avec un organe d'introduction à proximité de l'extrémité proximale.

2. Cathéter selon la revendication 1, comprenant une trifurcation (15) à l'extrémité proximale avec une sortie du côté distal auquel le corps de base (2) a été relié et trois sorties séparées du côté proximal pour le canal de guidage de câble, le canal de pression (5) et le canal de drainage respectivement.

3. Cathéter suivant la revendication 2, dans lequel la sortie du canal de guidage du câble a été pourvue d'une valve hémostatique (45).

4. Cathéter suivant la revendication 2 ou 3, dans lequel les sorties du canal de pression et du canal de drainage sont pourvues d'adaptateurs Luer-Lock (46, 47).

5. Cathéter suivant les revendications 3 et 4, dans lequel la valve hémostatique (45) et les adaptateurs Luer-Lock (46, 47) ont été intégrés dans la trifurcation.

6. Cathéter suivant l'une quelconque des revendications 2 à 5, dans lequel la trifurcation (40) a été fabriquée de telle façon que le canal de drainage s'étend en ligne droite et le canal de guidage de câble et le canal de pression s'étendent latéralement, de façon parallèle l'un à l'autre suivant un angle oblique à partir du canal de drainage.

7. Cathéter suivant l'une quelconque des revendications 2 à 6, dans lequel un canal de liaison (21) entre le canal de pression (5) et le canal de drainage (3) a été formé dans la trifurcation (15) qui débouche dans le canal de drainage (3) au moyen d'une buse de pulvérisation (22) dirigée dans la direction proximale.

8. Cathéter suivant l'une quelconque des revendications précédentes, dans lequel le corps de base (2) a été réalisé dans une section proximale relativement rigide, et ledit au moins un organe séparé en forme de tube consiste en un élément continu.
